# EUROPEAN PATENT APPLICATION

(11) **EP 3 459 430 A1**
(43) Date of publication of application: **27.03.2019**
(21) Application number: 17799350.8
(22) Date of filing: 15.05.2017
(51) Int. Cl.: A61B 1/04, A61B 1/00, A61B 1/045, A61B 1/07, G02B 23/24

(54) **ENDOSCOPE DEVICE**

(30) Priority: 17.05.2016 JP 2016098729; 21.06.2016 JP 2016123049
(71) Applicant: Kairos Co., Ltd., Tokyo 1050014 (JP)
(72) Inventor: CHIBA, Toshio, Tokyo 101-0062 (JP); YAMASHITA, Hiromasa, Tokyo 101-0062 (JP); TANIOKA, Kenkichi, Tokyo 162-0814 (JP); TAKAHASHI, Akira, Kokubunji-shi Tokyo 185-0014 (JP)
(74) Representative: Müller-Boré & Partner Patentanwälte PartG mbB
(86) International application number: PCT/JP2017/018255
(87) International publication number: WO 2017/199926

(57) **Abstract**

A high-resolution endoscope device that is reduced in size and weight is provided. The endoscope device (100) according to the present invention comprises an insertion unit (110) configured to be inserted into a body cavity and guide light from an object, an illumination device (120) attached to the insertion unit (110) and illuminating the object, and an imaging device (130) having an imaging element (131) provided with 8K-level or higher-level pixels arranged in a matrix form. The imaging element (131) receives light reflected from the object and guided by the insertion unit (110) and outputs imaging signals of the object. The insertion unit (110) and the illumination device (120) are able to be attached to the imaging device (130). The imaging device (130) is able to be grasped and carried by a human hand. The imaging device (130) has a mounting part (135) that incorporates the imaging element (131) and a grasping part (136) that is grasped and carried by a human hand. The cross-sectional area of the mounting part (135) perpendicular to an optical axis is equal to or larger than the cross-sectional area of the grasping part (136) perpendicular to the optical axis.

## Description

### [Technical Field]

The present invention relates to an endoscope device and particularly to an endoscope device using an 8K high-resolution video technique.

### [Background Art]

Endoscopes have been widely used which are configured to insert an elongate insertion unit into a body cavity and capture images inside the body cavity for less-invasive surgery. Recently, the probability of Japanese people getting cancer is about 50%, and the endoscopes tend to be widely used.

On the other hand, with the development of communication technology, image processing technology, and optical technology, a high-resolution video technique called 8K is being put into practical use. Changes from 2K to 4K and further to 8K will lead to qualitative technical innovation not only in mere increase of memory capacity but also in the field of medical devices using endoscopes and in the field of less-invasive surgery. Application of 8K high-resolution video technique to endoscope devices allows for recognition, for example, of fine surgical sutures, fine diseased sites of organs, and boundaries between organs/tissues as well as observation at the cell level. This can increase the reliability and certainty of surgery, and progress in the medical technology will be expected. That is, identification of diseased sites of organs is enhanced to reduce the possibility that the other sites than the diseased sites will be unexpectedly injured. Moreover, the field of view in operation can be expanded to make the surgery easy even in a wide operative area, which may be advantageous in checking the positions of surgical instruments and avoiding interference between the surgical instruments. Furthermore, large-screen observation is available, so that all the persons concerned in the surgery can share the same image and communication will be smooth (see Non-Patent Document 1). Thus, the use of 8K high-resolution video technique has great expansibility.

### [Prior Art Document(s)]

### [Non-Patent Document(s)]

[Non-Patent Document 1] Hiromasa Yamashita, "Application of 8K television technique to endoscopic surgery," Committee of first investigation on optical technique held in 2015, published on May 25, 2015

### [Summary of the Invention]

### [Problems to be solved by the Invention]

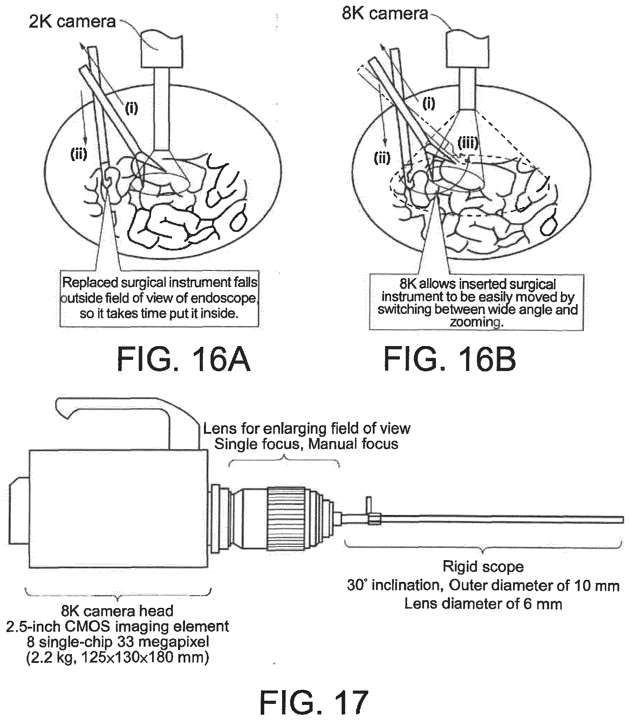

However, when using the 8K high-resolution video technique (also simply referred to as "8K," hereinafter), points to be modified are being found. FIG. 17 illustrates an example of a conventional 8K imaging device. Up to now the 8K video technique has been developed in the broadcasting field, so it has been found that the device is too large and heavy to use it in a state of being attached to an endoscope device. That is, the device is too heavy even when it is held by two surgical supporters. Moreover, in less-invasive surgery using an endoscope device, a delicate change of the imaging site is manually controlled, and in order to operate the endoscope device, it is preferred to miniaturize the grasping part so that it fits in a hand.

The present invention has been made in view of the above circumstances and an object of the present invention is to provide a high-resolution endoscope device that is reduced in size and weight.

### [Means for solving the Problems]

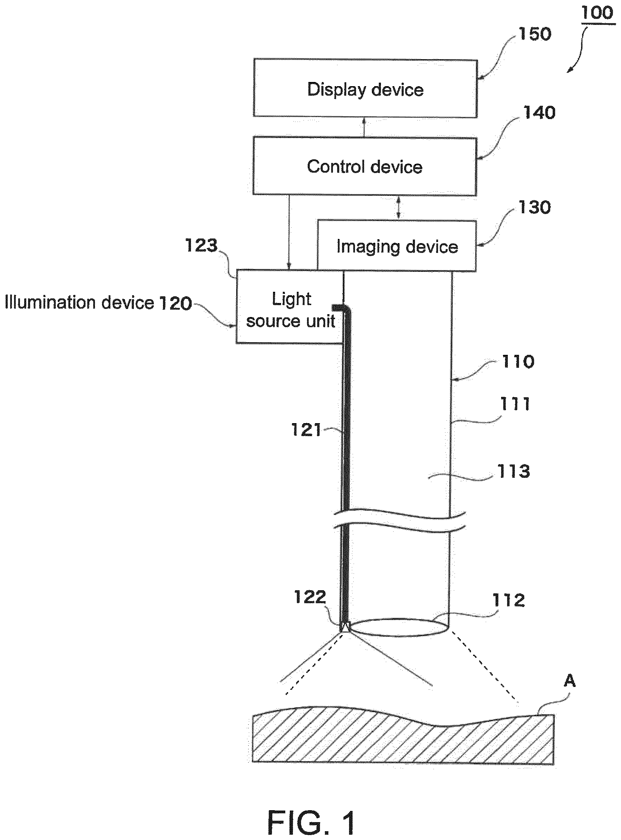

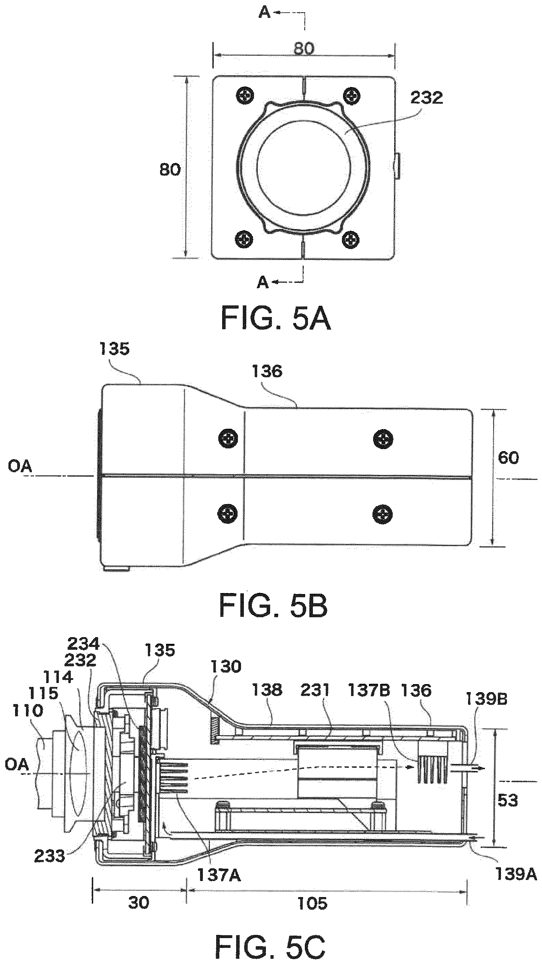

To solve the above problems, according to a first aspect of the present invention, an endoscope device 100 is provided, for example, as illustrated in FIG. 1. The endoscope device 100 comprises an insertion unit 110 configured to be inserted into a body cavity and guide light from an object A, an illumination device 120 attached to the insertion unit 110 and illuminating the object A, and an imaging device 130 having an imaging element 131 equipped with 8K-level or higher-level pixels arranged in a matrix form. The imaging element 131 receives light reflected from the object A and guided by the insertion unit 110 and outputs imaging signals of the object A. The insertion unit 110 and the illumination device 120 are able to be attached to the imaging device 130. The imaging device 130 is able to be grasped and carried by a human hand. The imaging device has a mounting part 135 that incorporates the imaging element 131 and a grasping part 136 that is grasped and carried by a human hand. The cross-sectional area of the mounting part 135 perpendicular to an optical axis OA (dashed-dotted line in FIG. 5) is equal to or larger than the cross-sectional area of the grasping part 136 perpendicular to the optical axis OA.

Here, "8K level" or "8K equivalent" refers to a degree of resolution equivalent to a high-definition resolution image that can be realized with 8K (7680×4320 pixels). In real world, however, resolutions that exceed the 4K resolution (3840x2160 pixels) may be used. It is therefore assumed herein that "8K level" or "8K equivalent" refers to cases in which resolutions exceed the 6K resolution (specifically, the number of pixels of one frame is more than 20,000,000). Because of the term "8K-level or higher-level," the number of pixels of 8K resolution (7680x4320 pixels) or more may be used. A typical cross-sectional area for 8K may preferably be 80±10 mm×80±10 mm at the mounting part 135 and 60±10 mm×53±10 mm at the grasping part 136. An unduly large cross-sectional area at the mounting part 135 may increase the weight while an unduly small cross-sectional area at the mounting part 135 may cause unclear images due to insufficient pixel pitch. An unduly large or small cross-sectional area at the grasping part 136 may make it difficult to grasp the grasping part 136. Because of the term "8K or higher," the cross-sectional area of the mounting part 135 may be larger. According to such a configuration, it is possible to provide an endoscope device including the high-resolution imaging device 130 which is reduced in size and weight. It is to be noted that, also in a case in which a flange or the like having a cross-sectional area comparable to that of the mounting part 135 is attached to the rear of the grasping part 136 for some reasons, such as a reason that the optical axis is set horizontal when the imaging device 130 is placed on a horizontal plane, for example, the cross-sectional area of the mounting part 135 is to be compared with the cross-sectional area of the grasping part 136.

According to a second aspect of the present invention, an endoscope device 100 is provided, for example, as illustrated in FIG. 1. The endoscope device 100 comprises an insertion unit 110 configured to be inserted into a body cavity and guide light from an object A, an illumination device 120 attached to the insertion unit 110 and illuminating the object A, and an imaging device 130 having an imaging element 131 equipped with 8K-level or higher-level pixels arranged in a matrix form. The imaging element 131 receives light reflected from the object A and guided by the insertion unit 110 and outputs imaging signals of the object A. The insertion unit 110 and the illumination device 120 are able to be attached to the imaging device 130. The imaging device 130 is able to be grasped and carried by a human hand. The imaging element has a pitch of 2.8 µm or more and 3.8 µm or less.

The pitch of pixels (pixel pitch) P is appropriately 2.8 to 3.8 µm. An unduly small pitch will cause interference to blur the images. An unduly large pitch will lead to a large-size substrate, which may be disadvantageous in terms of the volume, weight, speed, and the like. The pixel pitch P may further appropriately be 3.0 to 3.5 µm.

In the first aspect of the present invention, as illustrated in FIG. 1, for example, the endoscope device 100 according to a third aspect of the present invention may be arranged such that the illumination device 120, the imaging device 130, and a control device 140 are configured as separate devices and the imaging device 130 has a weight of 500 g or less.

Here, the control device 140 may include a control unit 141, an image processing unit 142, and a storage unit 143. These units are disposed outside the imaging device 130 to reduce the weight of the imaging device 130. In addition or alternatively, the housing of the imaging device 130 may be produced using a lightweight metal or a lightweight plastic (such as FRP) thereby to reduce the weight of the imaging device 130. In an alternative embodiment, not all the portions of the control device 140 may be disposed outside the imaging device 140 (130), that is, one or more portions that are less likely to affect the weight may be left in the imaging device 140 (130). For example, it suffices that 90% or more as the weight of the control device 140 is disposed outside the imaging device 140 (130). As of this moment, the weight of a conventional lightweight endoscope device is 2.2 kg (see FIG. 17). According to this aspect of the present invention, the weight of the imaging device 130 can be reduced to 500 g or less due to the above weight saving in addition to the miniaturization according to the first aspect of the present invention. With such a configuration, the imaging device can be further reduced in weight as compared with the first aspect of the present invention.

In any one of the first to third aspects of the present invention, as illustrated in FIG. 1, for example, the endoscope device 100 according to a fourth aspect of the present invention may be arranged such that the insertion unit 110 has a lens system that includes an objective lens 112 providing a view angle of 80 degrees or more and a diffusion layer that diffuses light supplied from the illumination device 120 and outputs the diffused light to the object A.

Here, a wide view angle can be achieved by using a wide-angle lens as the objective lens. An angle of 80 to 180 degrees may be preferred as the 8K equivalent. Such a configuration allows the lens system to increase the view angle.

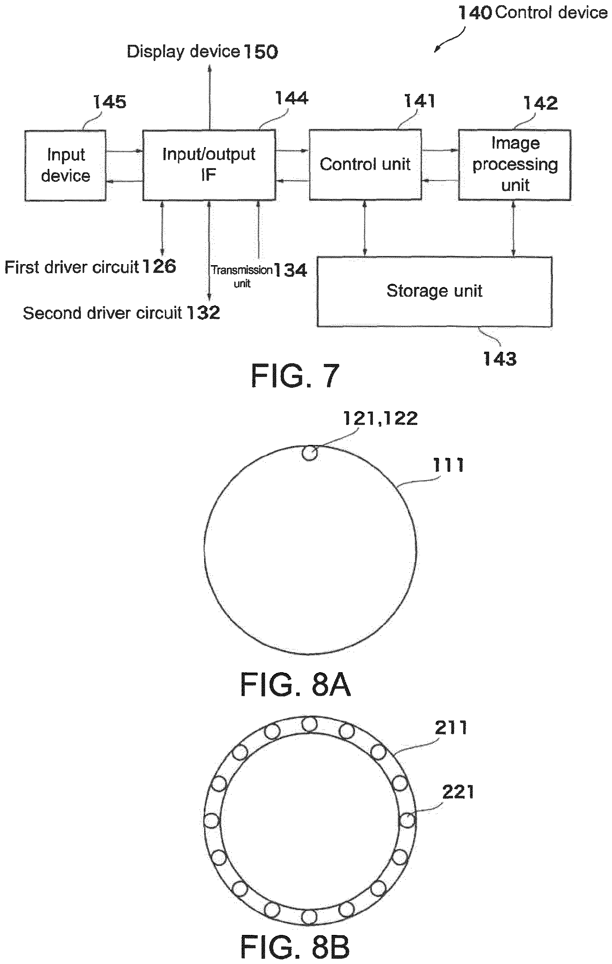

In any one of the first to fourth aspects of the present invention, as illustrated in FIG. 1 and FIG. 7, for example, the endoscope device 100 according to a fifth aspect of the present invention may be arranged such that the imaging device 130 has an A/D conversion unit that converts a pixel voltage into digital pixel data, and the endoscope device 130 may further comprise: a control device 140 having a storage unit 143 that stores the pixel data provided from the imaging device 130 and an image processing unit 142 that creates frame data from the pixel data, processes the frame data, and uses digital zooming for magnification adjustment on the frame data; and a display device 150 that displays the frame data created by the image processing unit 142 on a large screen.

Here, the digital zooming (electronic zooming) is to cut out and enlarge a part of the captured image. High resolution can be obtained by 8K even in details, so the enlargement does not degrade the definition. It is also possible to vary the magnification in the vertical and horizontal directions. The term "large screen" refers to a monitor screen of 30 inches or more. Such a configuration allows the high resolution to be obtained even when the view angle is increased by the digital zooming.

To solve the above problems, according to a sixth aspect of the present invention, an endoscope device 100 is provided, for example, as illustrated in FIG. 1. The endoscope device 100 comprises an insertion unit 110 configured to be inserted into a body cavity and guide light from an object A, an illumination device 120 attached to the insertion unit 110 and illuminating the object A, and an imaging device 130 having an imaging element 131 equipped with 8K-level or higher-level pixels arranged in a matrix form. The imaging element 131 receives light reflected from the object A and guided by the insertion unit 110 and outputs imaging signals of the object A. The insertion unit 110 and the illumination device 120 are able to be attached to the imaging device 130. The imaging device 130 is able to be grasped and carried by a human hand. The endoscope device 100 further comprises: a control device 140 having a storage unit 143 that stores pixel data provided from the imaging device 130 and an image processing unit 142 that creates frame data from the pixel data, processes the frame data, and uses digital zooming for magnification adjustment on the frame data; and a display device 150 that displays the frame data created by the image processing unit 142 on a large screen. The object A can be focused with a distance of 1 to 15 cm between a distal end of the insertion unit 110 and the object A.

Here, the distance between the distal end of the- insertion unit 110 and the object A is preferably 1 to 15 cm and more preferably 8 to 12 cm from the viewpoint of the size of the surgical space and the visibility in the surgical area. Such a configuration allows the distance between the distal end of the insertion unit 110 and the object A to be increased, and a wide surgical space can therefore be created between the distal end of the insertion unit 110 and the object A. It is thus possible to display not only images of the surgical portion but also images of a wide range of areas including the surroundings of the surgical portion. Moreover, less-invasive single-incision surgery can be performed to reduce the collision between surgical instruments. Furthermore, the use of digital zooming at 8K enables observation of microscopic areas with high definition. Zoom-in and zoom-out operations allow microscopic areas and wide regions to be switched for display or displayed simultaneously. This can shorten the replacement time for a surgical instrument. In addition, display on a large screen allows all the persons concerned in the surgery to share the images during the surgery. Moreover, no loupe is necessary. Thus, the creation of a wide surgical space, the digital zooming, and the large screen display significantly change the surgical environment. This improves the medical care to be highly reliable, safe, and secure.

In the endoscope device according to a seventh aspect of the present invention, the insertion unit 110 has a tubular part 111 that has a length of 10 to 20 cm. Such a configuration allows the length of the insertion unit 110 to be short and it is therefore possible to mitigate the image fluctuation due to hand movement of an operator who grasps the imaging device. Moreover, the number of relay lenses can be small and the light attenuation and aberration can be reduced, thus resulting in a bright lens system. Clear images can therefore be obtained.

### [Effect of the Invention]

According to the present invention, a high-resolution endoscope device that is reduced in size and weight can be obtained.

### [Brief Description of Drawings]

FIG. 1 is a diagram illustrating the configuration of an endoscope device according to Example 1.
FIG. 2 is a diagram illustrating the detailed configuration of an illumination device and an imaging device.
FIG. 3 is a diagram for describing the pixel pitch of an imaging element.
FIG. 4 is an external view of the imaging device.
FIG. 5 is a set of diagrams illustrating the board arrangement and the housing dimensions of the imaging device, wherein FIG. 5(a) is a front view, FIG. 5(b) is a plan view, and FIG. 5(c) is a cross-sectional view along line A-A.
FIG. 6 is a set of assembly diagrams of components of the imaging device, wherein FIG. 6(a) is an exploded view and FIG. 6(b) is a perspective view of the completed body after assembly.
FIG. 7 is a block diagram illustrating the detailed configuration of a control device.
FIG. 8 is a set of diagrams illustrating arrangements of optical fibers in tubular parts of insertion units, wherein FIG. 8(a) illustrates a configuration in which the number of an optical fiber is one and FIG. 8(b) illustrates a configuration in which a plurality of optical fibers is arranged along the circumference.
FIG. 9 is a set of views illustrating comparison examples (part 1) of endoscopic images between 8K and 2K, wherein FIG. 9(a) is a view illustrating an endoscopic image (captured image), FIG. 9(b) is a view illustrating a 256-times magnified image at 2K, and FIG. 9(c) is a view illustrating a 256-times magnified image at 8K.
FIG. 10 is a diagram for describing a novel surgical space using an 8K endoscope.
FIG. 11 is a diagram schematically illustrating a configuration example of an 8K endoscope system.
FIG. 12 is a set of views illustrating comparison examples (part 2) of endoscopic images between 8K and 2K, wherein FIG. 12(a) illustrates 2K endoscopic images (a captured image and its partially enlarged view) and FIG. 12(b) illustrates 8K endoscopic images (a captured image and its partially enlarged view).
FIG. 13 is a set of diagrams for describing the appearance of boundaries (adhesive interfaces) between organs/tissues, wherein FIG. 13(a) is a diagram for explaining that the adhesive interface can be identified and FIG. 13(b) is a diagram for explaining that the adhesive interface can be separated.
FIG. 14 is a set of views comparing the field of view of a 2K endoscope and the field of view of an 8K endoscope, wherein FIG. 14(a) is a view illustrating an example of an 8K endoscopic image and FIG. 14(b) is a view illustrating an example of a 2K endoscopic image.
FIG. 15 is a diagram for describing the superiority of an 8K endoscope in single-incision surgery.
FIG. 16 is a set of diagrams for describing the superiority of an 8K endoscope when replacing a surgical instrument, wherein FIG. 16(a) is a diagram for describing replacement of a surgical instrument using a 2K endoscope and FIG. 16(b) is a diagram for describing replacement of a surgical instrument using an 8K endoscope.
FIG. 17 is a diagram illustrating an example of a conventional imaging device.

### [Mode(s) for Carrying out the Invention]

Hereinafter, the endoscope device according to one or more embodiments of the present invention will be described in detail with reference to the drawings.

### <Example 1>

FIG. 1 illustrates the configuration of an endoscope device according to the present example. The endoscope device 100 according to the present example, which is a rigid scope that is primarily used as a laparoscope or a luminal scope, comprises an insertion unit 110, an illumination device 120, an imaging device 130, a control device 140, and a display device 150.

The insertion unit 110 is an elongate member that is inserted into a body cavity of a person under test or the like. The insertion unit 110 has a tubular part 111, an objective lens 112, and a hollow light guide region 113.

The tubular part 111 is a member configured such that a metal material such as a stainless steel material, a hard resin material, or the like is formed into a cylindrical or elliptical cylindrical shape having, for example, a diameter of 8 mm to 9 mm. The illumination device 120 is detachably attached to a side surface in the vicinity of the base end of the tubular part 111 and the imaging device 130 is detachably attached to the base end portion of the tubular part 111.

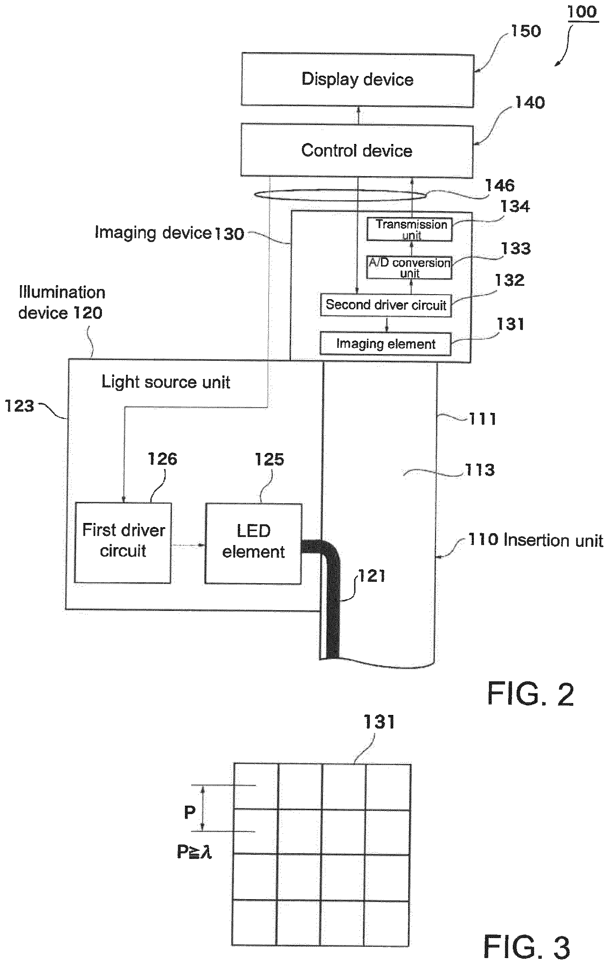

The objective lens 112 is a light guide means that introduces light emitted from the illumination device 120 and reflected by an object A in the body cavity. The objective lens 112 is composed, for example, of a wide-angle lens. This allows a wide view angle to be obtained. The view angle is preferably 80 degrees to 180 degrees or more. The objective lens 112 is disposed so as to be exposed from the distal end surface of the insertion unit 110. The objective lens 112 converges the reflected light from the object A and forms an image of the object A on an imaging surface on which an imaging element 131 (see FIG. 2) of the imaging device 130 is disposed, via the hollow light guide region 113. The side surface of the objective lens 112 is fixed to the inner wall surface in the vicinity of the distal end portion of the tubular part 111 using an adhesive or the like, and the distal end surface of the insertion unit 110 is thus sealed.

The hollow light guide region 113 is a space arranged between the base end portion and distal end portion of the cylindrical part 111 and serves as a light guide means that guides the light having passed through the objective lens 112 to the imaging device 130.

FIG. 2 illustrates a detailed configuration of the illumination device 120 and imaging device 130 of the endoscope device 100. The illumination device 120 has an optical fiber 121, a diffusion layer 122, and a light source unit 123. The optical fiber 121 is led out from the light source unit 123 and fixed to the inner surface of the tubular part 111 with an adhesive or the like and extends to the diffusion layer 122 at the distal end portion of the tubular or cylindrical part.

The diffusion layer 122 (see FIG. 1) diffuses and outputs the light which is supplied from the light source unit 123 via the optical fiber 121. The diffusion layer 122 is composed, for example, of a diffusing plate or a diffusing lens that diffuses the incident light and outputs the diffused light. The diffusion layer 122 is provided to diffuse the light so as to cover the view angle obtained by the wide-angle objective lens 112.

The light source unit 123 supplies light for illuminating the object A to the base end portion of the optical fiber 121. The light source unit 123 comprises a light emitting diode (LED) element 125 and a first driver circuit 126. A xenon lamp can be used as substitute for the LED element 125, but in this case the light source unit 123 is provided separately from the insertion unit 110, and the illumination light is introduced into the insertion unit 110 via a long cord.

The LED element 125 incorporates elements that emit light of three colors of red (R), green (G), and blue (B) and irradiates the incident end of the optical fiber 121 with white light obtained by color mixing.

The first driver circuit 126 drives the LED element 125 under the control by the control device 140. The first driver circuit 126 performs dimming control of the LED element 125 by PWM control or the like under the control by the control device 140.

The imaging device 130, which is detachably attached to the base end portion of the insertion unit 110, captures an image of the object A with the incident light having passed through the hollow light guide region 113 of the cylindrical part 111 and supplies the captured image to the control device 140. More specifically, the imaging device 130 has an imaging element 131, a second driver circuit 132, an AID conversion unit 133, and a transmission unit 134.

FIG. 3 is a diagram for describing a pixel pitch of the imaging element. The imaging element 131 is composed of a so-called 8K color image sensor, that is, a color image sensor of 7680×4320 pixels. According to the 8K endoscope device 100, high-definition captured images can be obtained.

However, true resolution (image denseness) of 8K cannot necessarily be achieved on the display device (display) 150 by simply setting the number of pixels of an image sensor to 8K (7680×4320 pixels). To truly realize a resolution of 8K, it is required that "the size of pixels be large." If the size of pixels of an image sensor is unduly small, the captured images cannot be resolved due to the diffraction limit of light, resulting in blurred images. When applied to an endoscope, a large-sized image sensor may be difficult to use without any modification because the diameter of a built-in lens of the endoscope is very small due to the limitation that the endoscope has to be inserted into a body cavity.

It is conceivable to enlarge the diameter of a light beam guided in the endoscope to the entire area of the image sensor using a magnifying lens. However, the higher the magnification (the farther the focal point distance), the larger the area of an image circle on the screen increases, but the range of an operative field in which the reflected light can be obtained narrows. This may lead to a problem in that the amount of light (photons) received by the image sensor decreases to make the image dark. This problem was able to be solved in 8K because the sensitivity of the image sensor was quadrupled and the brightness of a liquid crystal monitor was enhanced.

To realize a resolution of 8K, the pitch P of pixels of the imaging element 131 is set to a size equal to or larger than the diffraction limit of primary light used for illumination of the object A. Specifically, the pitch P is set to a value larger than a reference wavelength A corresponding to the wavelength of the illumination light emitted from the diffusion layer 122, that is, the wavelength of the emission light of the LED element 125. When the illumination light includes light having a plurality of wavelengths, the reference wavelength λ means the wavelength of light having the longest wavelength among the three primary colors of light which constitute the illumination light, that is, the wavelength of the primary component of red light. That is, the reference wavelength λ means the wavelength with the largest energy in the spectral region corresponding to red.

As the aperture (f value) of a lens system is increased, the brightness is enhanced, but the resolution deteriorates. As the aperture is reduced, the resolution is enhanced, but the brightness deteriorates. It has thus been found that, in 8K, the aperture (f value) is appropriately 10 to 16 and the pitch of pixels (pixel pitch) P is appropriately 2.8 to 3.8 µm. An unduly small pitch will cause interference to blur the images. An unduly large pitch will lead to a large-size substrate, which may be disadvantageous in terms of the volume, weight, speed, and the like. The pixel pitch P may further appropriately be 3.0 to 3.5 µm. Assuming that the pixel pitch P is 2.8 to 3.8 µm, the size of the imaging element 131 is about 20 to 30 mm×12 to 18 mm. When this is surrounded by a frame part 234 of the substrate on which the imaging element 131 is mounted and a circular eyepiece mount part 114 and further surrounded by a rectangular frame part and a housing 138, the size of the mounting part 135 which incorporates the imaging element 131 is, for example, 80 mmx80 mm×30 mm. On the other hand, the size of the grasping part 136 is, for example, 60 mm×53 mm×105 mm

(see FIG. 5) in view of easiness of grasping and operation. In 8K or higher, therefore, considering the variations of ±10 mm in the vertical and horizontal dimensions, the cross-sectional area of the mounting part 135 perpendicular to the optical axis is typically equal to or larger than the cross-sectional area of the grasping part 136 perpendicular to the optical axis. Again, the mounting part 135 incorporates the imaging element 131 and the grasping part 136 is grasped and carried by a human hand. When the mounting part is circular, the size of the mounting part is, for example, 70 mmϕ×30 mm. Nevertheless, the cross-sectional area of the mounting part, which incorporates the imaging element 131, perpendicular to the optical axis is equal to or larger than the cross-sectional area of the grasping part, which is grasped and carried by a human hand, perpendicular to the optical axis.

The color of a fine region can be recorded in one pixel because the number of pixels is large. For example, in 8K, it is possible to identify fine sutures of 20 µm. (Pixels are visible with the naked eye in 2K, but not visible in 8K.) The number of pixels of 8K (about 33 million) is 16 times that of 2K (about 2 million). In the display device 150 (see FIG. 1), the number of pixels is represented by (field of view (monitor area))×(pixel density). For example, if the field of view is quadrupled that of 2K, the pixel density will also be quadrupled that of 2K. Assuming that the view angle when viewing the monitor screen is 30 degrees in 2K, 60 degrees in 4K, and 100 degrees in 8K, the reality is saturated at 100 degrees, so 8K is sufficient if the reality is required.

The imaging element 131 may comprise pixels equivalent to or larger than 8K. In real world, even with the number of elements of 8K or less, clear images can be obtained as compared with 4K, so such elements may be put on sale or the like with indication of 8K. It is therefore assumed that "8K equivalent" refers to the number of pixels of 6K or more.

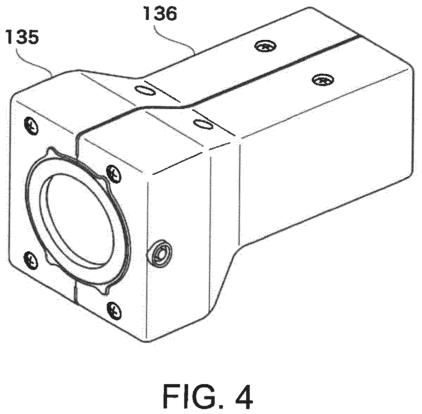

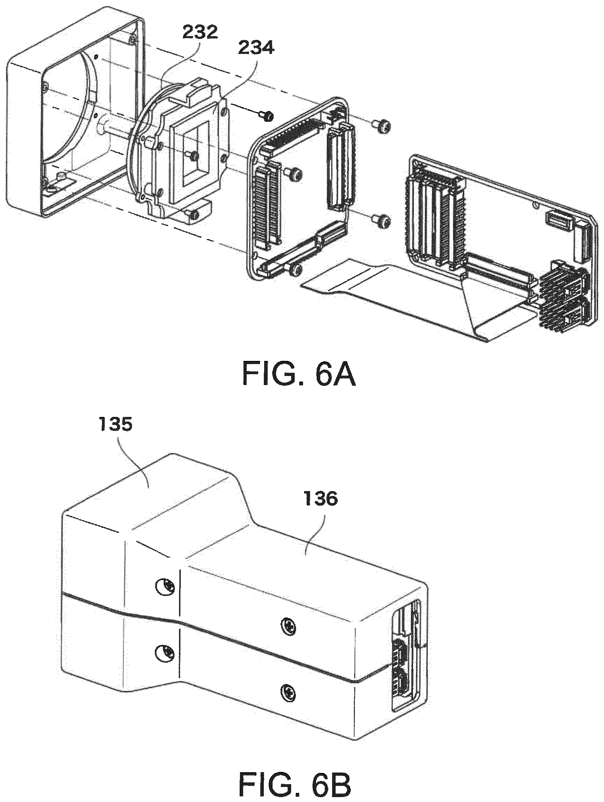

FIG. 4 is an external view of the imaging device 130, FIG. 5 is a set of diagrams illustrating the board arrangement and the housing dimensions of the imaging device 130, and FIG. 6 is a set of assembly diagrams of components. FIG. 5(a) is a front view, FIG. 5(b) is a plan view, and FIG. 5(c) is a cross-sectional view along line A-A. FIG. 6(a) is an exploded view and FIG. 6(b) is a perspective view of the completed body after assembly. The mounting part 135 has a size of about 80 mmx80 mmx30 mm, and a diaphragm 233 and an imaging substrate (on which the imaging element 131 is mounted) are mounted on the mounting part 135. The eyepiece mount part 114 on which an eyepiece 115 is mounted is attached to the mounting part 135, and the insertion unit 110 is attached to the eyepiece mount part 114. The grasping part 136 has a size of about 60 mm×53 mm×105 mm, which is a size that allows the grasping part 136 to be grasped in a hand. In order to mount the imaging substrate having a size of (pixel pitch)×(number of pixels) required for 8K, the mounting part 135 has a cross-sectional area equal to or larger than the cross-sectional area of the grasping part 136. In FIG. 5, reference numeral 137A denotes a Peltier element that cools the substrate on which the imaging device 131 is mounted, reference numeral 137B denotes a Peltier element (which may not be necessary) that cools a control substrate 231 (which may not be necessary) left in the imaging device 130, and reference numeral 232 denotes a circular frame body that is in contact with the frame body of the eyepiece mount part 114 (the same size) and connected to the diaphragm 233. In FIG. 6, reference numeral 232 denotes the circular frame body and reference numeral 234 denotes a frame body of the substrate on which the imaging device 131 is mounted.

To reduce the size and weight, the housing 138 is formed, for example, using a lightweight metal (such as Al) or FRP (e.g. a nylon powder-processed one), and a primary part of the control device 140, the illumination device 120, and the cooling fan, which would be incorporated in a conventional imaging device, were able to be put outside thereby to achieve a weight of 500 g or less. This allows for easy operation and transportation and greatly improves the reliability of surgery.

Referring again to FIG. 2, the second driver circuit 132 controls the start and end of exposure of the imaging element 131 under the control by the control device 140 and reads out the voltage signal of each pixel (pixel voltage). The A/D conversion unit 133 converts the pixel voltage read out from the imaging element 131 by the second driver circuit 132 into digital data (image data) and outputs the digital data to the transmission unit 133 (134). The transmission unit 134 outputs the luminance data, which is output from the A/D conversion unit 133, to the control device 140.

FIG. 7 is a block diagram illustrating a detailed configuration of the control device 140. The control device 140, which controls the endoscope device 100 as a whole, comprises a control unit 141, an image processing unit 142, a storage unit 143, an input/output interface (IF) 144, and an input device 145.

The control unit 141, which is composed of a central processing unit (CPU), memories, and other necessary components, controls the storage unit 143 to store the luminance data transmitted from the transmission unit 134, controls the image processing unit 142 to process the image data, and controls the display device 150 to display the processed image data. The control unit 141 further controls the first driver circuit 126 and the second driver circuit 132.

The image processing unit 142, which is composed of an image processor and other necessary components, processes the image data stored in the storage unit 143 under the control by the control unit 141, reproduces the image data of each frame (frame data), and re-accumulates the reproduced image data in the storage unit 143. The image processing unit 142 also performs various image processes on the image data of each frame unit stored in the storage unit 143. For example, the image processing unit 142 performs a scaling process for enlarging/reducing each image frame at an arbitrary magnification.

Digital zooming is used for the scaling process. Images are not blurred even when enlarged by the digital zooming because clear images are accumulated in the storage unit 143. This allows images in a wide field of view to be clearly displayed, which make it possible to provide a surgical space with a wide field of view. Moreover, when the digital zooming is used together with image processing (a sharpening process), clearer images can be obtained, such as by emphasizing the contrast between the diseased sites and other sites for representation.

According to the endoscope device 100 of the present embodiment, the LED element 125 with which large energy can be obtained is used as the light source of the illumination device 120. This allows bright illumination light and therefore a bright image to be obtained.

FIG. 8 illustrates an arrangement of the optical fiber 121 in the cylindrical part 111 of the insertion unit 110. FIG. 8(a) illustrates a configuration in which the number of the optical fiber 121 according to the present example is one while FIG. 8(b) illustrates a configuration in which a plurality of conventional optical fibers 221 is arranged along the cylindrical part 211.

The illumination light is guided by the optical fiber 121 disposed on the inner wall of the cylindrical part 111 and, therefore, the space in the hollow light guide part 113 of the cylindrical part 111 can be effectively used for guiding the light from the object A. This will be more specifically described. As illustrated in FIG. 8(b), a scheme of arranging optical fibers 221 on the circumference of a cylindrical part 211 is known as a form of the illumination of an endoscope. According to this scheme, the space inside the cylindrical part 211 is occupied by the optical fibers 221 for illumination. This narrows the optical path for the image of an object and makes it difficult to project a large image on the imaging surface of the imaging element 131. In contrast, in the endoscope device 100, as illustrated in FIG. 8(a), the hollow light guide part 113 of the cylindrical part 111 can be widely utilized for guiding the light from the object A. In the case of the endoscope device 100, effective utilization of the hollow light guide part 113 is very advantageous because the outer diameter of the insertion unit is limited.

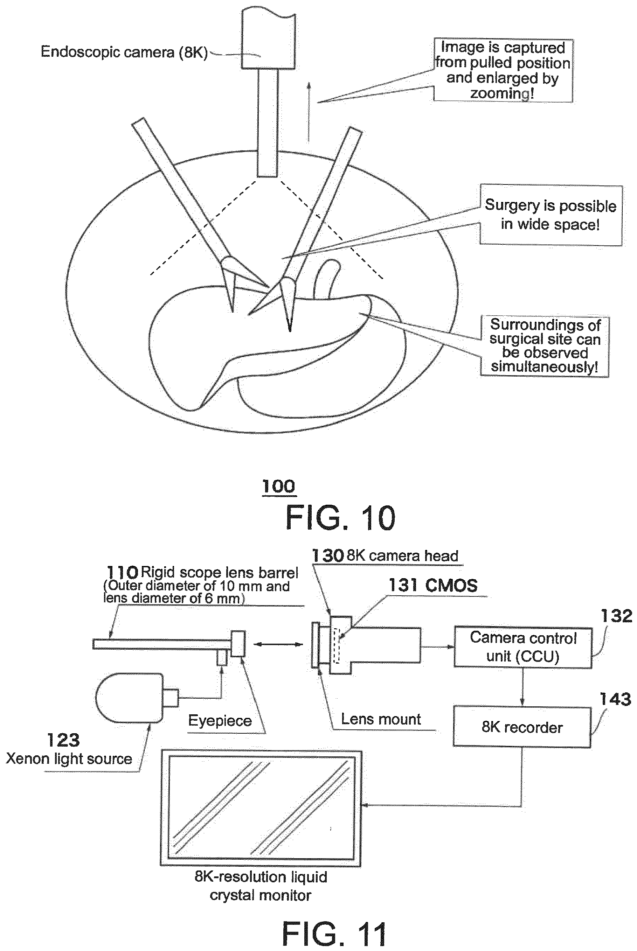

FIG. 11 schematically illustrates the concept of a configuration example of an 8K endoscope system. The primary parts of the 8K endoscope system 100 are the insertion unit 110 and the imaging device 130. The insertion unit 110 is composed, for example, of a rigid scope lens barrel having an outer diameter of 10 mm and a lens diameter of 6 mm, and the imaging device 130 is composed, for example, of an 8K camera head having a CMOS as the imaging element 131. A xenon light source is used as the light source unit 123 of the illumination device 120, and the eyepiece of the insertion unit 110 and the lens mount of the imaging device 130 are coupled. The image data captured by the imaging device 130 is stored in an 8K recorder as the storage unit 143 of the control device 140 via a camera control unit as the second driver circuit 132 and displayed on a liquid crystal monitor (exhibiting 8K resolution) 150 as the display device 150 via the control unit 141.

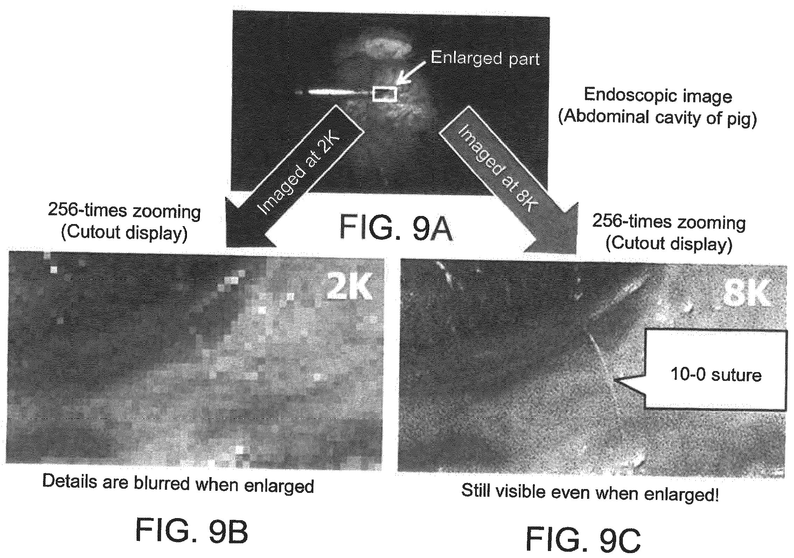

FIG. 9 illustrates comparison examples (part 1) of endoscopic images between 8K and 2K. FIG. 9(a) illustrates a 2K endoscopic image (captured image), FIG. 9(b) illustrates a 256-times magnified image (partial cutout) at 2K, and FIG. 9(c) illustrates a 256-times magnified image at 8K (partial cutout). These endoscopic images show the abdominal cavity of a pig. In the 2K image, details may be blurred and unrecognizable when enlarged, but in the 8K image, for example, 10-0 sutures (0.020 to 0.029 mm in diameter) can be recognized.

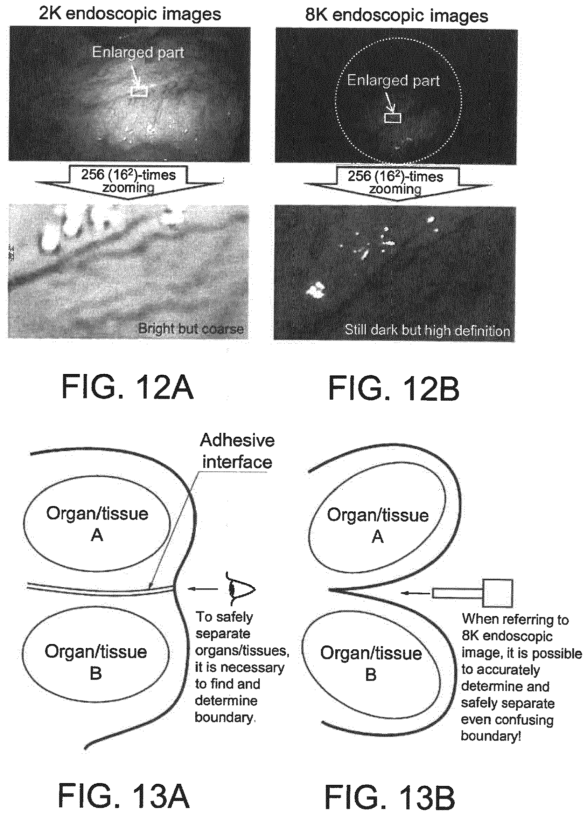

FIG. 12 illustrates comparison examples (part 2) of endoscopic images between 8K and 2K. FIG. 12(a) illustrates 2K endoscopic images (a captured image and its partially enlarged view) and FIG. 12(b) illustrates 8K endoscopic images (a captured image and its partially enlarged view). The large intestine surface blood vessels are blurred in the 2K images, but in the 8K images they are viewed with high-definition resolution.

FIG. 13 is a set of diagrams for describing the appearance of boundaries (adhesive interfaces) between organs/tissues. FIG. 13(a) is a diagram for explaining that the adhesive interface can be identified and FIG. 13(b) is a diagram for explaining that the adhesive interface can be separated. To safely separate the adhesive interface between organs/tissues, it is necessary to find and determine the boundary. When referring to an 8K endoscopic image, it is possible to accurately determine and separate even a confusing boundary. It is also possible to distinguish normal sites and abnormal sites such as cancer.

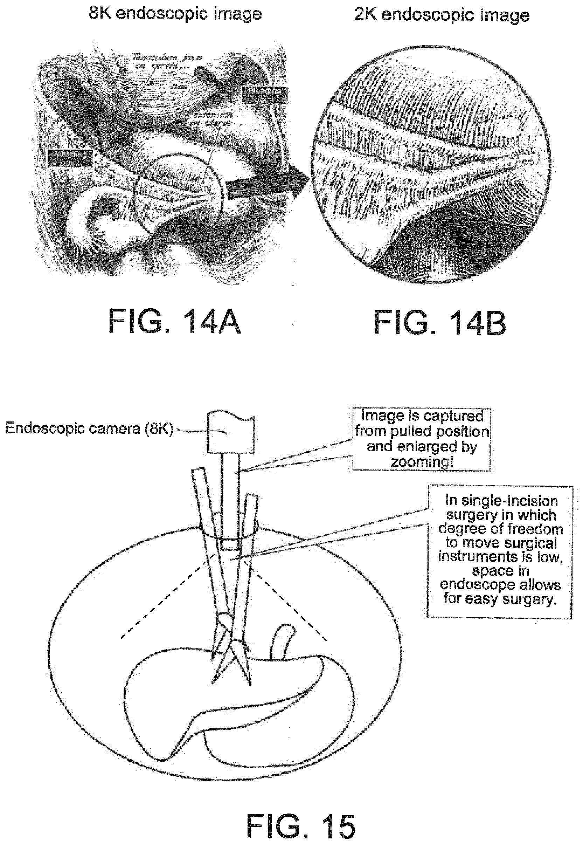

FIG. 14 is a set of views comparing the field of view of a 2K endoscope and the field of view of an 8K endoscope. FIG. 14(a) is a view illustrating an example of an 8K endoscopic image and FIG. 14(b) is a view illustrating an example of a 2K endoscopic image. The conventional 2K endoscope allows only a narrow central region to be viewed, so even when a bleeding point exists around the central region, it will be missed. However, fortunately, the 8K endoscope allows not only the central region (surgical region) but also a wide range including the surroundings of the central region to be switched for display or displayed simultaneously; therefore, bleeding points can be found around the central region, which will make the practitioner to feel safe.

FIG. 10 is a diagram for describing a novel surgical space using an 8K endoscope. In an 8K image, the image can be enlarged with a widened view angle while keeping the sharpness. It is therefore possible, for example, to grasp the distal end position of the surgical instrument while zooming out, position the distal end of the surgical instrument through the operation of taking in and out the surgical instrument, and perform observation while zooming in. Moreover, if the view angle is wide, a fixed camera is placed at the pulled position to capture an image, which can then be enlarged by digital zooming to display a wide surgical space. This can realize a surgical space with a wide field of view, which can be well adapted to surgery with a wide range of operation. For example, it is not necessary to move the camera, interference of surgical instruments can be avoided, and other advantages can be obtained. Furthermore, it is also possible to display an enlarged image obtained by zooming up the operation site together with the image of a wide field of view before zooming up.

FIG. 15 is a diagram for describing the superiority of an 8K endoscope in single-incision surgery. The use of an 8K endoscopic camera allows the object A to be imaged from a position separate from the object A and it is thus easy to avoid collision of surgical instruments. Single-incision surgery is evaluated as less-invasive (patient's burden is small), but the degree of freedom to move surgical instruments is low. The 8K endoscope allows images to be captured from a pulled position, and the surgery can be performed while observing the captured image enlarged by digital zooming. The surgery is thus easy because the endoscope can be separated from the surgical position.

FIG. 16 is a set of diagrams for describing the superiority of an 8K endoscope when replacing a surgical instrument. FIG. 16(a) is a diagram for describing replacement of a surgical instrument using a 2K endoscope and FIG. 16(b) is a diagram for describing replacement of a surgical instrument using an 8K endoscope. The field of view is narrow in the 2K endoscope; therefore, when the surgical instrument used until then is taken out (i) and a surgical instrument to be replaced is taken into the surgical space (ii), the latter may fall outside the field of view of the endoscope, so that it sometimes takes time to put the replaced surgical instrument inside the field of view. When the 8K endoscopic camera is used, after the surgical instrument is replaced by another one (i), (ii), it is possible to zoom out in a wide range to look for the replaced surgical instrument, put it inside the field of view, and then zoom in to perform the surgery (iii). Thus, replacement and movement of the surgical instrument can be facilitated to shorten the duration of surgery.

Here, the surgical space will be described. For example, the length of the tubular part 111 of the insertion unit 110 is 10 to 20 cm (conventionally 20 to 30 cm), the invasion length into a body cavity is 0 to 150 mm (conventionally 100 to 200 mm), and the focal distance of the objective lens system is 10 to 150 mm. Observation at close range is possible, and the object can be focused in a wide range. The surgical space is inflated by gas injection. Conventionally, because of optical observation, the range in which the object can be focused and clear images can be obtained has been narrow. In contrast, according to one or more embodiments of the present invention, the captured image can be observed by digital zooming and it is therefore possible to clearly observe the image of the object A even at a relatively long distance. Observation by digital zooming and the shortened invasion length into the body cavity make it possible to take a wide surgical space. For example, the distance between the distal end of the insertion unit 110 and the object A (the height of the surgical space) can be 50 to 150 mm above the object. When the invasion length into the body cavity is set to 0 to 30 mm and the distance between the distal end of the insertion unit 110 and the object A (the height of the surgical space) is set to 80 to 120 mm above the object, the surgical space can be widened and the image can be clearly observed, which may be preferred. As will be understood, observation at close range may also be necessary, in which case observation at 10 to 50 mm is possible. Moreover, the use of digital zooming makes a loupe unnecessary, thus facilitating the surgery. Furthermore, the length of the insertion unit 110 is short and it is therefore possible to mitigate the image fluctuation due to hand movement of an operator who grasps the imaging device. In addition, when the length of the insertion unit 110 is short, the number of relay lenses can be small and the light attenuation and aberration can be reduced, thus resulting in a bright lens system. Clear images can therefore be obtained.

A wide surgical space allows for less-invasive (patient's burden is small) and single-incision (only one incision) surgery. Collision of surgical instruments such as surgical scalpels and forcipes can be avoided, and when a surgical instrument is replaced, it is possible to zoom out to search for the surgical instrument and correct its position. This can shorten the duration of surgery. Thus, when the 8K image display technique and the zoom-in and zoom-out technique are used, the reliability and safety of surgery are enhanced, the duration of surgery can be shortened, and the surgical environment greatly changes. In this way, the creation of a wide surgical space, the digital zooming, and the large screen display significantly change the surgical environment. This improves the medical care to be highly reliable, safe, and secure.

The storage unit 143 stores the operation program for the control unit 141, the operation program for the image processing unit 142, the image data received from the transmission unit 134, the frame data reproduced and processed by the image processing unit 142, etc.

The input/output IF 144 serves as an interface for transmission and reception of data between the control unit 141 and an external device. The input device 145, which is composed of a keyboard, a mouse, buttons, a touch panel, and other necessary components, supplies an instruction from the user to the control unit 141 via the input/output IF 144.

The display device 150, which is composed of a liquid crystal display device or the like having a display pixel number corresponding to 8K, displays an operation screen, a captured image, a processed image, etc. under the control by the control device 140. The 8K allows a large screen monitor to be used as the display device. For example, a large screen monitor of 30 inches or more is used because the storage unit 143 stores pixels of 7680x4320 pixels. Even a large screen monitor allows for natural viewing. All the persons concerned in the surgery can therefore share the images on one or more large screens to achieve smooth communications. (Surgeons can also view a branched image from the imaging device.)

Unlike the cameras for television broadcasting, the endoscope is used in a dedicated facility. The imaging device 130 attached to the insertion unit 110 is therefore connected to the control device 140 via cables 146 (see FIG. 2) of about several meters. The control device 140 and the display device 150 are placed on a table or the like. The illumination device 120, the imaging device 130, and the control device 140 are separated from one another. Accordingly, the structures attached to the insertion unit 110 (the insertion unit 110, the imaging device 130, and the illumination device 120) are reduced in weight and size, and handling of the insertion unit 110 is thus relatively easy. Moreover, the cables 146 connecting the illumination device 120 and the imaging device 130 to the control device 140 are at most 1 to 10 m in an operation room, and such cables are different from those for a broadcasting site, which may exceed several 100 m in some cases. Thus, signal deterioration due to the cables 146 is small and there is almost no adverse effect by separation.

With reference to FIG. 5(c), cooling means 137 of the imaging device 130 will be described. When the operation of the imaging device 130 is continued, heat is generated. When the sensitivity of the image sensor which constitutes the imaging element 131 is increased, the amount of heat generation further increases. When the temperature of the imaging device 130 rises, noise components to the video signal increase to lower the S/N ratio of the signal, and the image quality of the image displayed on the monitor screen deteriorates. For the above reasons, the cooling means 137 is provided.

In the present example, a Peltier element as the cooling means 137 is provided inside the housing 138, and heat dissipation from the imaging element 131 is performed on the heat generating side of the Peltier element 137A. That is, the housing 138 is provided with an air intake port 139A that takes in the external air and an air exhaust port 139B that discharges the internal air of the housing 138, and the housing 138 has a sealed structure except the air intake port 139A and the air exhaust port 139B (no ethylene oxide gas for disinfection enters the housing 138). One end of an on-off valve member (not illustrated) is connected and fixed to each of the air intake port 139A and the air exhaust port 139B. The air intake port 139A is driven by a negative pressure source (not illustrated), and the air in the housing 138 is discharged from the air exhaust port 139B side by the negative pressure suction force of the negative pressure source. This forms an airflow in which the external air is taken in from the air intake port 139A, and a cooling flow path is thus formed. The heat generating side of the Peltier element 137A is exposed to that airflow, and heat exchange takes place with the air of the external air temperature. The Peltier element 137B dissipates the heat of the substrate 139C left in the imaging device. As a result, even when heat is generated in the housing 138 due to the driving of the imaging device 130, components are efficiently cooled to suppress the temperature rise of the imaging device 130. Thus, the imaging device 130 is maintained at a high S/N ratio state to reduce the noise to the video signal, and a high-quality image is displayed on the monitor. The length of an air exhaust pipe may have to be 5 m or more so that the dust mixed in the exhaust air does not enter the operation space.

The operation of the endoscope device 100 having the above configuration will then be described. When using the endoscope 100, the user (practitioner) operates the input device 145 to input an instruction to turn on the endoscope device 100. In response to this instruction, the control unit 141 turns on the first driver circuit 126 and the second driver circuit 132. The first driver circuit 126 turns on the light source (such as the xenon lamp or the LED element 125), while the second driver circuit 132 starts imaging with the imaging element 131. The white light output from the LED element 125 is guided through the optical fiber 121 and diffused by the diffusion layer 122 for irradiation.

The imaging element 131 captures a video footage through the objective lens 121 (112) and the hollow light guide part 123 (113). It has been found that the pitch P of pixels is preferably 2.8 to 3.8 µm from the relationship between the aperture (f value) of the lens system and the pitch P of pixels of the imaging element 131, so a pitch P of pixels within that range was used. This allows for acquisition of bright and high-resolution images.

The second driver circuit 132 sequentially reads out the pixel voltages of respective pixels from the imaging element 131, and the read out pixel voltages are converted by the A/D conversion unit 133 into digital image data, which are sequentially transmitted from the transmission unit 134 to the control device 140 via the cables 146.

The control unit 141 of the control device 140 sequentially receives the transmitted image data via the input/output IF 144 and in turn stores the image data in the storage unit 143.

Under the control by the control unit 141, the image processing unit 142 processes the image data stored in the storage unit 143 to reproduce the frame data and may perform additional processing thereon as appropriate.

The control unit 141 appropriately reads out the frame data stored in the storage unit 143 and supplies the frame data to the display device 150 via the input/output IF 144 for display.

The user (practitioner) inserts the insertion unit 110 into the body cavity while confirming the display on the display device 150. When the insertion unit 110 is inserted in the body cavity, the object A is illuminated with light from the diffusion layer 122 and the imaging device 131 captures an image of the object A, which is displayed on the display device 150.

As described above, according to the present example, a high-resolution endoscope device that is reduced in size and weight can be provided.

### <Example 2>

The present example will be described as an example in which buttons for control are disposed on the housing 138 of the imaging device 130. The buttons are used for control of the display position on the display device 150, control of the scaling, adjustment of the focal point of the lens system of the insertion unit 110, adjustment of the diaphragm, etc. The buttons are useful because such control and adjustment can be performed at hand. Other device configurations are the same as those in Example 1, and a high-resolution endoscope device that is reduced in size and weight can be provided as in Example 1.

### <Example 3>

The present example will be described as an example in which a database is utilized. If medical diagnostic data in past times are accumulated in the storage unit 143, such accumulated data can be utilized for operations and data analysis. For example, it is possible to compare images that vary over time, to compare the health status and the abnormal status, and to compare other necessary matters. This can develop the medical and diagnostic techniques, thus leading to enhancement of the reliability. Other device configurations are the same as those in Example 1, and a high-resolution endoscope device that is reduced in size and weight can be provided as in Example 1.

One or more embodiments of the present invention have been heretofore described, but the present invention is not limited to the above-described embodiments. It should be appreciated that various modifications can be made to the embodiments without departing from the spirit of the present invention.

For example, the present examples have been described as those in which one optical fiber is attached to the insertion unit 110, but two or more optical fibers may be employed to increase the illumination light and the two or more optical fibers may be arranged along the circumferential part 115 so as to surround the entire circumference. Moreover, the present examples have been described as those in which one objective lens 112 constitutes a lens system that receives, in the insertion unit 110, the reflected light from the object and transmits the light to the imaging device 130, but another configuration may also be employed in which the reflected light from the object is transmitted to the imaging device 130 via two or more relay lenses. Furthermore, the present examples have been described as those in which the objective lens 112 of the insertion unit 110 is installed horizontally, but it may be installed with an inclination. When the insertion unit 110 is installed with an inclination, the insertion unit can be rotated to allow for observation of a wide range. In the image processing, images that have varied over time may be superimposed for comparison. In this case, translucent images may be used to make it easier to perceive changes in diseased sites due to the passage of time. In addition, appropriate modifications are possible, within an appropriate range, for the shape, size, and weight of each part of the endoscope device, the screen dimensions of the imaging element and image monitor, etc.

### [Industrial Applicability]

The present invention may be utilized for an endoscope device used for less-invasive surgery.

### [Description of Reference Numerals]

- 100: Endoscope device
- 110: Insertion unit
- 111: Tubular part
- 112: Objective lens
- 113: Hollow light guide region
- 114: Eyepiece mount
- 115: Eyepiece
- 120: Illumination device
- 121: Optical fiber
- 122: Diffusion layer
- 123: Light source unit
- 125: LED element
- 126: First driver circuit
- 130: Imaging device
- 131: Imaging element
- 132: Second driver circuit
- 133: A/D conversion unit
- 134: Transmission unit
- 135: Mounting part
- 136: Grasping part
- 137, 137A, 137B: Cooling means (Peltier element)
- 138: Housing
- 139A: Air intake port
- 139B: Air exhaust port
- 140: Control device
- 141: Control unit
- 142: Image processing unit
- 143: Storage unit
- 144: Input/output IF
- 145: Input device
- 146: Cables
- 150: Display device
- 211: Circumferential part
- 221: Optical fibers
- 231: Substrate
- 232: Circular frame body
- 233: Diaphragm
- 234: Frame part of substrate on which imaging element is mounted
- A: Object
- OA: Optical axis

## Claims

1. An endoscope device comprising:
an insertion unit configured to be inserted into a body cavity and guide light from an object;
an illumination device attached to the insertion unit and illuminating the object; and
an imaging device having an imaging element equipped with 8K-level or higher-level pixels arranged in a matrix form, the imaging element receiving light reflected from the object and guided by the insertion unit and outputting imaging signals of the object, the insertion unit and the illumination device being able to be attached to the imaging device, the imaging device being able to be grasped and carried by a human hand,
wherein the imaging device has a mounting part that incorporates the imaging element and a grasping part that is grasped and carried by the human hand, and a cross-sectional area of the mounting part perpendicular to an optical axis is equal to or larger than a cross-sectional area of the grasping part perpendicular to the optical axis.

2. An endoscope device comprising:
an insertion unit configured to be inserted into a body cavity and guide light from an object;
an illumination device attached to the insertion unit and illuminating the object; and
an imaging device having an imaging element equipped with 8K-level or higher-level pixels arranged in a matrix form, the imaging element receiving light reflected from the object and guided by the insertion unit and outputting imaging signals of the object, the insertion unit and the illumination device being able to be attached to the imaging device, the imaging device being able to be grasped and carried by a human hand,
wherein the imaging element has a pitch of 2.8 µm or more and 3.8 µm or less.

3. The endoscope device as recited in claim 1 or claim 2, wherein the illumination device, the imaging device, and a control device are configured as separate devices, and the imaging device has a weight of 500 g or less.

4. The endoscope device as recited in any one of claim 1 to claim 3, wherein the insertion unit has a lens system that includes an objective lens providing a view angle of 80 degrees or more and a diffusion layer that diffuses light supplied from the illumination device and outputs the diffused light to the object.

5. The endoscope device as recited in any one of claim 1 to claim 4, wherein the imaging device has an A/D conversion unit that converts a pixel voltage into pixel data, and the endoscope device further comprises: a control device having a storage unit that stores the pixel data provided from the imaging device and an image processing unit that creates frame data from the pixel data, processes the frame data, and uses digital zooming for magnification adjustment on the frame data; and a display device that displays the frame data created by the image processing unit on a large screen.

6. An endoscope device comprising:
an insertion unit configured to be inserted into a body cavity and guide light from an object;
an illumination device attached to the insertion unit and illuminating the object;
an imaging device having an imaging element equipped with 8K-level or higher-level pixels arranged in a matrix form, the imaging element receiving light reflected from the object and guided by the insertion unit and outputting imaging signals of the object, the insertion unit and the illumination device being able to be attached to the imaging device, the imaging device being able to be grasped and carried by a human hand;
a control device having a storage unit that stores pixel data provided from the imaging device and an image processing unit that creates frame data from the pixel data, processes the frame data, and uses digital zooming for magnification adjustment on the frame data; and
a display device that displays the frame data created by the image processing unit on a large screen,
wherein the object can be focused with a distance of 1 to 15 cm between a distal end of the insertion unit and the object.

7. The endoscope device as recited in claim 6, wherein a tubular part of the insertion unit has a length of 10 to 20 cm.
